# EUROPÄISCHE PATENTSCHRIFT

(11) **EP 1 895 018 B1**
(45) Veröffentlichungstag und Bekanntmachung des Hinweises auf die Patenterteilung: **02.05.2012**
(21) Anmeldenummer: 07016602.0
(22) Anmeldetag: 24.08.2007
(51) Int. Cl.: C12Q 1/70

(54) **Verfahren und Mittel zum Nachweis von humanen Papillomaviren**
Method and device for proving human papillomaviruses
Procédé et moyen de mise en évidence de papillomavirus humains

(30) Priorität: 28.08.2006 DE 102006041970
(43) Veröffentlichungstag der Anmeldung: 05.03.2008
(73) Patentinhaber: Genome Identification Diagnostics GmbH, 72479 Strassberg (DE)
(72) Erfinder: Weilbach, Alfons, 72074 Tübingen (DE)
(74) Vertreter: Patentanwälte Ruff, Wilhelm, Beier, Dauster & Partner

(56) Entgegenhaltungen:
- EP-A- 0 301 968
- EP-A- 0 425 995
- EP-A- 1 302 550
- WO-A-00/50645
- WO-A-02/103050
- WO-A-2005/056839
- US-A1- 2001 010 899
- TIEBEN L M ET AL: "DETECTION OF CUTANEOUS AND GENITAL HPV TYPES IN CLINICAL SAMPLES BY PCR USING CONSENSUS PRIMERS" JOURNAL OF VIROLOGICAL METHODS, AMSTERDAM, NL, Bd. 2/3, Nr. 42, Mai 1993 (1993-05), Seiten 265-279, XP008004426 ISSN: 0166-0934
- HARNISH D G ET AL: "Evaluation of human papillomavirus-consensus primers for HPV detection by the polymerase chain reaction" MOLECULAR AND CELLULAR PROBES, ACADEMIC PRESS, LONDON, GB, Bd. 13, Nr. 1, Februar 1999 (1999-02), Seiten 9-21, XP004441532 ISSN: 0890-8508
- CAMPIONE-PICCARDO J ET AL: "A highly conserved nucleotide string shared by all genomes of human papillomaviruses." VIRUS GENES OCT 1991, Bd. 5, Nr. 4, Oktober 1991 (1991-10), Seiten 349-357, XP009092925 ISSN: 0920-8569
- DATABASE EMBL [Online] 22. September 2006 (2006-09-22), "Sequence 23 from Patent WO2006094238." XP002472617 gefunden im EBI accession no. EMBL:CS415169 Database accession no. CS415169 & WO 2006/094238 A (ISIS PHARMACEUTICALS INC [US]; SAMPATH RANGARAJAN [US]) 8. September 2006 (2006-09-08)
- DATABASE Geneseq [Online] 14. Januar 2000 (2000-01-14), "HPV16 E1 ORF PCR primer 2." XP002472618 gefunden im EBI accession no. GSN:AAZ23742 Database accession no. AAZ23742 & WO 99/50424 A (STANLEY MARGARET ANNE [GB]; ZHANG WEI [GB]) 7. Oktober 1999 (1999-10-07)

## Beschreibung

Die vorliegende Erfindung betrifft ein PCR-System zum Nachweis von Infektionen mit humanen Papillomaviren (HPV) und ein entsprechendes Verfahren. Weiterhin betrifft die vorliegende Erfindung einen Kit zum Nachweis von Infektionen mit HPV.

Humane Papillomaviren (HPV) zeigen in ihrem Genomaufbau eine einheitliche Struktur. Es sind mittlerweile mehr als 150 verschiedene humane Papillomaviren bekannt. Eine Bezeichnung der HPV erfolgt numerisch fortlaufend, eingeteilt nach ihrer Desoxyribonukleinsäure (DNA)-Sequenz. Die humanen Papillomaviren lassen sich in drei Gruppen gliedern: mucokutane (Warzen bildung), Epidermodysplasie assoziierte (kutane Neoplasien) und anogenitale HPV (Kondylome, Anal- und Zervixkarzinome).

Die Papillomaviren sind weltweit verbreitet. Infektionen mit diesen Viren sind art- und weitgehend organspezifisch. HPV weisen ein ringförmiges Genom auf; das sämtliche viralen Gene codiert. Eine Unterteilung der Gene erfolgt nach ihren Expressionsphasen in frühe Gene, sogenannte "early genes", E1 bis E8, und späte Gene, sogenannte "late genes", L1 und L2. Proteine der frühen Gene besitzen unter anderem regulatorische Funktionen und beeinflussen das Zellwachstum infizierter menschlicher Zellen. E6- und E7-Genprodukte haben transformierende Eigenschaften und sind für die onkogene Potenz von Virustypen, insbesondere der Virustypen HPV 16 und HPV 18, verantwortlich.

Bei der Infektion mit dem humanen Papillomavirus handelt es sich um eine Kontaktinfektion. Es gibt eine Vielzahl von klinischen Erscheinungen, die sehr vom Virustyp, dem betroffenen Gewebe und der Resistenzsituation des befallenen Organismus abhängen. Aktive, klinisch relevante Infektionen weisen eine erhöhte Wahrscheinlichkeit einer Integration von viraler DNA in das menschliche Genom auf. Damit erhöht sich die Wahrscheinlichkeit einer tumorösen Entartung von Zellen. Weitere Cofaktoren wie Immunsuppression, HIV-Infektion, Rauchen, Chlamydien- und Herpesinfektionen sowie genetische Faktoren scheinen eine Rolle bei der Unterdrückung oder Elimination der HPV-Infektion zu spielen.

Es sind mehr als 20 HPV-Typen mit invasiven Zervixkarzinomen assoziiert. Die HPV-Typen 6, 11, 30, 42, 43 und 44 und andere werden aufgrund ihres geringen onkogenen Risikos als sogenannte Niedrigrisiko-HPV-Typen ("low risk") eingestuft. Hingegen weisen Hochrisiko-HPV-Typen, insbesondere die HPV-Typen 16, 18, 45 und 56, ein hohes onkogenes Risiko auf ("high risk"). Die Viren lassen sich sowohl in leichten, wie auch in mäßig bis schweren Dysplasien (zervikale intraepitheliale Neoplasien, eingestuft in die Klassen CIN 1 bis 3) sowie in invasiven Karzinomen, insbesondere in Zervixkarzinomen, nachweisen. Es gilt als

gesichert, dass HPV notwendige Risikofaktoren für die Entstehung von Krebserkrankungen, insbesondere von Gebärmutterhalskrebs, darstellen. Insbesondere sind die HPV-Typen 16 und 18 mit invasiven Zervixkarzinomen assoziiert.

Spezifische antivirale Therapien, insbesondere durch Impfungen, könnten in naher Zukunft erhältlich werden, es wird allerdings ein längerer Zeitraum vergehen, bis derartige Therapien sich durchgesetzt haben werden. Befallene Haut- bzw. Schleimhautbereiche werden auch weiterhin je nach Größe durch Laser, Kryotherapie oder Exzision behandelt werden. Bei malignen Formen von Erkrankungen im Genitalbereich ist in der Regel eine operative Entfernung (Resektion) notwendig. Ein effektives Früherkennungsprogramm bleibt daher weiterhin unverzichtbar.

Frühstadien von Zervixkarzinomen werden derzeit hauptsächlich durch Entnahme eines Zellabstrichs (zervikaler Abstrich, auch als Papanicolaou-Abstrich, kurz Pap-Abstrich, bezeichnet), in Verbindung mit einer Kolposkopie erfasst. Obwohl die Einführung zytologischer Vorsorgeprogramme zu einer Abnahme der Inzidenz von Zervixkarzinomen geführt hat, konnte bisher keine weitere signifikante Senkung erreicht werden. Zytologische Verfahren sind mit einem relativ großen subjektiven Fehler behaftet, nicht standardisiert und können keine befriedigenden Ergebnisse über die Weiterentwicklung einzelner Läsionen liefern. Anzucht und kulturelle Vermehrung von HPV sind technisch sehr aufwendig und scheiden daher als Nachweis aus. Immunhistochemische Verfahren sind nicht sensitiv genug, serologische Untersuchungen mit Nachweis HPVspezifischer Antikörper haben für die Diagnose keine Bedeutung, da selbst bei Patientinnen mit Zervixkarzinom nur in der Hälfte aller Fälle Antikörper nachweisbar sind.

Um insbesondere bei einem auffälligen zytologischen Befund, eine Aussage über eine mögliche Entwicklung maligner Tumore treffen zu können, werden Nachweisverfahren für virale Nukleinsäuren in klinischen Gewebeproben eingesetzt. Insbesondere Verfahren zur Unterscheidung individueller Typen von Niedrig- und Hoch-Risiko-HPV-Infektionen. Ein signalverstärkendes Hybridisierungsverfahren der Fa. Digene ist als Hybrid-capture-microplate (HCM)-Methode bekannt. Die HCM-Methode verwendet HPV-spezifische RNA-Sequenzen als Hybridisierungssonden. Der Nachweis von RNA/DNA-Hybriden erfolgt in Mikroplatten mittels enzymkonjugierter Antikörper in einem photometrischen Verfahren. Einige Hochrisiko-Typen können jedoch nicht erfasst werden und Kreuzreaktionen können zwischen beiden HPV-Klassen auftreten. Dabei ist besonders zu berücksichtigen, wie wichtig falsch-negative oder falschpositive Ergebnisse für die Psyche von Patienten sind. Derartige Ergebnisse sollten daher möglichst vermieden werden.

Viele im Stand der Technik bekannte Verfahren zum Nachweis viraler Nukleinsäuren in klinischen Gewebeproben beruhen auf einer Amplifikation von HPV-DNA. Im Polymerasekettenreaktionsverfahren (polymerase chain reaction, PCR) wird mit Hilfe spezifischer Primer die Amplifikation der DNA typspezifisch durchgeführt.

Die europäische Patentanmeldung EP 05009276 der Anmelderin und die PCT Anmeldung WO 2005/056839 offenbaren Kitsund Verfahren mit Mitteln zum Nachweis verschiedener HPV-Typen. Es werden diesbezügliche Primer und Oligonukleotidsonden beschrieben, als Methode für die Amplifikation wird vorzugsweise die PCR aufgeführt.

Ein erheblicher Nachteil bekannter kommerzieller Verfahren zum Nach-weis und zur Typisierung von HPV-Genotypen besteht vor allem darin, dass gering konservierte HPV-DNA-Bereiche als Vorlage für Primer und Oligonukleotidsonden eingesetzt werden. Dies gilt insbesondere für Sonden und Primer aus L1-Bereichen. Darauf aufbauende Nachweisverfahren weisen bedingt durch eine hohe Basenvariabilität in diesen Bereichen nur eine geringe Spezifität ihrer Sonden und Primer auf. Hierdurch können falsch-negative Testresultate bedingt sein.

Somit besteht fortwährend ein medizinischer Bedarf an schnellen und einfachen Verfahren zum sicheren Nachweis von verschiedenen HPV-Typen.

Es ist daher Aufgabe der vorliegenden Erfindung, ein unkompliziertes Nachweisverfahren für virale Nukleinsäuren, insbesondere aus HPV-DNA-Bereichen, sowie Mittel dafür, und auch ein Kit zur Durchführung des Verfahrens, bereitzustellen, so dass eine optimale Sicherheit für Nachweisergebnisse gewährleisten ist.

Die vorliegende Erfindung löst das ihr zugrunde liegende technische Problem durch die Bereitstellung eines PCR Systems, Verfahens und Kits wie den Ansprüchen 1-10 definiert.

Im Rahmen dieser Erfindung sollen die Begriffe Oligonukleotidsonden, Oligonukleotide und Gensonden gleichwertig verstanden werden.

Zum in-vitro Nachweis einer viralen Infektion mit einzelnen, insbesondere auch mehreren, HPV-Typen, ist es zweckmäßig, eine Amplifikation von HPV-DNA durchzuführen. In einer bevorzugten Ausführungsform der Erfindung wird zur DNA-Amplifikation eine Polymerasekettenreaktion (polymerase chain reaction, PCR) durchgeführt. Bei der PCR handelt es sich um eine Methode, Nukleinsäuren oder bestimmte Abschnitte von Nukleinsäuren in vitro gezielt zu vervielfältigen, ohne einen lebenden Organismus zu verwenden. Die PCR gliedert sich in Zyklen aufeinander folgender Denaturierungs-, Hybridisierungs- und Elongations-Schritte. Im Denaturierungsschritt wird doppelsträngige DNA thermisch unter Aufspaltung von Wasserstoffbrückenbindungen in Einzelstränge geteilt, die im Weiteren als Matrizen dienen. Im Hybridisierungsschritt lagern sich unter Temperaturabsenkung die Primer an die Matrizen an (Annealing). Daraufhin erfolgt im Elongations-Schritt mittels DNA-Polymerasen und freien Nukleotideinheiten eine Neusynthese von DNA-Abschnitten. Durch die Wahl entsprechender Primer, vorzugsweise von Primer-Paaren, werden die zu synthetisierenden DNA-Abschnitte vorgegeben. Nach mehrmaliger Wiederholung, beispielsweise 20 - 40 Zyklen, ist eine Konzentration der DNA erreicht, die zum Nachweis geeignete ist. Nach elektrophoretischer Auftrennung eines auf diese Weise erhaltenen Amplifikats kann zum HPV-Nachweis beispielsweise eine sogenannte Southern-blot-Hybridisierung mittels Virustyp-spezifischer markierter Oligonukleotidsonden erfolgen, die aus einem jeweils zu untersuchenden HPV-DNA-Abschnitt stammen. Alternativ lässt sich beispielsweise eine Sequenzierung eines Amplifikats zur HPV-Typisierung durchführen. Im Falle eines Nachweises von RNA oder RNA-Abschnitten ist es notwendig, die RNA zuerst umzukopieren. Dies kann mittels einer RNAabhängigen DNA-Polymerase, einer sogenannten reversen Transkriptase, durchgeführt werden. Der weitere Verlauf gestaltet sich dann wie oben beschrieben.

Unter dem Begriff "PCR" sollen im Rahmen der hier vorliegenden Erfindung auch weitere für eine Nukleinsäureamplifikation geeignete PCR-Varianten oder PCR-ähnliche Varianten verstanden werden, insbesondere eine verschachtelte (nested) PCR, eine Ligase-Ketten-Reaktion (ligase chain reaction, LCR) oder im Falle von RNA als Ausgangsmaterial, eine Nucleic acid sequence based amplification (NASBA)-Reaktion, eine reverse Transkriptase (RT)-PCR oder eine inverse PCR.

In den Nukleotidsequenzen der vorliegenden Erfindung gilt, dass M gleich A oder C ist, R gleich A oder G ist, W gleich A oder T ist, S gleich C oder G ist, Y gleich C oder T ist, K gleich G oder T ist, V gleich A oder C oder G ist, H gleich A oder C oder T ist, D gleich A oder G oder T ist, B gleich C oder G oder T ist, N gleich G oder A oder T oder C ist. Ferner ist I gleichbedeutend mit Inosin.

In der vorliegenden Offenbarung ist es vorgesehen, dass die Amplifikate mit den Oligonukleotidsonden mit mindestens einer der Nukleotidsequenzen SEQ ID Nr. 1 bis 48 aus beiliegendem Sequenzprotokoll sowie deren komplementäre Sequenzen hybridisieren. Die Oligonukleotidsonden werden für die jeweiligen HPV-Typen spezifisch bereitgestellt. Insbesondere ist auch offenbart, dass die Amplifikate mit den Oligonukleotidsonden hybridisieren, die spezifisch an Nukleinsäuren binden, an die die Nukleinsäuren mit den Nukleotidsequenzen SEQ ID Nr. 1 bis 48 aus beiliegendem Sequenzprotokoll binden, und dass komplementäre Sequenzen der Oligonukleotide ein-geseht werden. Im Rahmen der hier vorliegenden Erfindung sollen auch veränderte und/oder modifizierte, insbesondere degenerierte, Oligonukleotide umfasst sein. Insbesondere können durch Verkürzung, Verlängerung, Substitution, Insertion und/oder Deletion die erfindungsgemäßen Oligonukleotide verändert und/oder modifiziert sein, wobei ihre Funktion vorzugsweise unverändert ist. Vorzugsweise können die im Rahmen der Beschreibung angegebenen Nukleotidsequenzen mit den SEQ ID Nr. 1 bis 48 aus beiliegendem Sequenzprotokoll sowie deren komplementäre Sequenzen eine Addition und/oder Deletion von 1 bis 10 Nukleotiden und/oder eine Substitution von 1 bis 3 Nukleotiden aufweisen. Es ist auch möglich, dass die Sequenzen Teil eines Polynukleotids sind. Unter "Polynukleotid" soll im Rahmen der vorliegenden Erfindung ein Oligonukleotid mit 100 oder mehr Nukleotideinheiten verstanden werden. Erfindungsgemäß umfassen die Oligonukleotidsonden vorzugsweise 24 bis 27 Nukleotideinheiten.

Bei den in der vorliegenden Offenbarung eingesetzten Nukleinsäuren handelt es sich um synthetische Oligonukleotide, die als Primer bezeichnet werden. Es werden sogenannte Forward- und/oder Reverse-Primer (auch als Backward-Primer bezeichnet) eingesetzt. Es ist offenbart, dass die PCR mit Primern mit mindestens einer der Nukleotidsequenzen SEQ ID Nr. 49 bis 64 aus dem beiliegenden Sequenzprotokoll durchgeführt wird. Es ist auch offenbart , die PCR mit Primern durchzuführen, deren Sequenzen komplementär zu den SEQ ID Nr. 49 bis 64 sind. Die Primer werden für jeweilige HPV-Typen spezifisch bereitgestellt. Es ist auch offenbart, dass die PCR mit Primern durchgeführt wird, die spezifisch an Nukleinsäuren binden, an die die Nukleinsäuren mit den Nukleotidsequenzen SEQ ID Nr. 49 bis 64 aus dem beiligenden Sequenzprotokoll binden. Es ist auch offenbart, die PCR mit zu den SEQ ID Nr. 49 bis 64 komplementären Sequenzen durchzuführen. Weiterhin können die Primer modifiziert, insbesondere degeneriert sein.

Insbesondere können durch Verkürzung, Verlängerung, Substitution, Insertion und/oder Deletion die erfindungsgemäßen Primer verändert und/oder modifiziert sein, wobei ihre Funktion vorzugsweise unverändert ist. Vorzugsweise können die im Rahmen der Beschreibung angegebenen Nukleotidsequenzen mit den SEQ ID Nr. 49 bis 64 aus beiliegendem Sequenzprotokoll sowie deren komplementäre Sequenzen eine Addition und/oder Deletion von 1 bis 10 Nukleotiden und/oder eine Substitution von 1 bis 18 Nukleotiden aufweisen. Die Primer, umfassen vorzugsweise 24 bis 35 Nukleotideinheiten.

Bei HPV-Infektionen kommt es häufig zu einer Integration des Virus in die DNA humaner Zellen. Dabei treten in vielen Fällen Deletionen in der HPV-DNA auf, so dass solche Bereiche für einen Nachweis dann nicht mehr zur Verfügung stehen. Weiterhin muss berücksichtigt werden, das nur solche HPV-DNA-Bereiche für die Amplifikation mit den Primern in Frage kommen, die überwiegend konserviert sind, so dass die Primer optimal an die HPV-DNA binden, vorzugsweise mit einer maximalen Zahl von Wasserstoffbrückenbindungen. Häufige Variationen, insbesondere in betreffenden Genbereichen, würden eine Vielzahl von Primern und/oder Oligonukleotidsonden erfordern, um alle gewünschten HPV-Typen, insbesondere Hochrisiko-HPV-Typen, zu erfassen. Überraschenderweise zeigte sich, dass die genannten Nukleotidsequenzen und/oder dazu komplementäre Sequenzen für Zielsequenzen aus einem bestimmten Genabschnitt des E1-Gens von HPV besonders vorteilhaft zum Nachweis und sogar zur Typisierung von HPV sind, insbesondere zum Nachweis und zur Typisierung von allen bekannten Hochrisiko-HPV-Typen, da das E1-Gen seltener von Deletionen betroffen ist als beispielsweise das E6-Gen.

In einer weiteren Ausführungsform werden Gemische von Primern bereitgestellt, vorzugsweise äquimolare Gemische von Primern, insbesondere Gemische von Primern mit mindestens den Nukleotidsequenzen SEQ ID Nr. 49 bis 64 aus beiliegendem Sequenzprotokoll sowie auch Nukleinsäuren, die spezifisch an Nukleinsäuren binden, an die die Nukleinsäuren mit den Nukleotidsequenzen SEQ ID Nr. 49 bis 64 aus beiliegendem Sequenzprotokoll binden.

Diese Maßnahme hat den Vorteil, dass in einer einzigen PCR alle nachweisbaren und gesuchten HPV-Typen amplifiziert und nachgewiesen werden können. Erfindungsgemäß umfassen die Gemische Forward- und/oder Reverse-Primer.

In einer weiteren Ausführungsform umfassen die Gemische Primer mit mindestens den zuvor genannten Nukleotidsequenzen, wobei mindestens ein Nukleotid durch Inosin substituiert ist.

Diese Maßnahme hat den Vorteil, dass Variationen im HPV-DNA-Bereich durch Einsatz von Primern, die Inosin als eine Art universeller Nukleotideinheit aufweisen, ausgeglichen werden. Inosin ist vorteilhaft, da es jede Base in seiner komplementären Position als Partner akzeptiert. Besonders vorteilhaft ist der Einsatz von Inosin in Primern, wenn Zielsequenzen in der HPV-DNA an bestimmten Positionen einen hohen Grad an Degeneration aufweisen, insbesondere dann, wenn alle 4 Basen-Typen an den betreffenden Positionen vorkommen können. Vorteilhafterweise verringert sich hierdurch die für den Nachweis notwendige Anzahl an Primern.

In einer weiteren bevorzugten Ausführungsform der vorliegenden Erfindung sind die Primer markiert, insbesondere mit einer Nicht-Eiweißverbindung, vorzugsweise mit Biotin.

Diese Maßnahmen haben den Vorteil, dass das mit markierten Primern synthetisierte Amplifikat durch mindestens eine Detektionsart nachgewiesen werden kann. Eine Markierung des Amplifikats kann unter Verwendung von markierten Primern und/oder markierten Nukleotideinheiten erfolgen. Als Markierungen kommen beispielsweise radioaktive Stoffe, insbesondere radioaktiver Phosphor und/oder radioaktives Iod, Fluoreszenz-Farbstoffe, insbesondere Fluorescein, Haptene, Antigene, eiweißfreie Verbindungen, insbesondere prosthetische Gruppen oder Glykoside, vorzugsweise Biotin oder Digoxygenin in Frage. Als Beispiele für Detektionsmethoden seien neben anderen spektroskopische, photometrische und/oder geeignete enzymatische Verstärkungsreaktionen genannt. Erfindungsgemäß ist es insbesondere vorgesehen, dass die Primer mit Biotin markiert sind.

Die vorliegende Erfindung löst das ihr zugrunde liegende technische Problem auch in dem eingangs genannten Verfahren zur Diagnose von Infektionen, insbesondere von Infektionen mit humanen Papillomaviren (HPV), wobei der DNA-Nachweis über spezifische Bereiche von HPV-DNA unter Einsatz von mindestens einer Oligonukleotidsonde mit einer der Nukleotidsequenzen gemäß der vorliegenden Erfindung und Primern mit mindestens einer der Nukleotidsequenzen gemäß der vorliegenden Erfindung vorgenommen wird.

Das Verfahren gemäß der vorliegenden Erfindung ist zur Diagnose von Infektionen vorgesehen. Insbesondere dient es zur Charakterisierung von HPV-Infektionen. So können HPV typisiert werden. Mit Vorteil können Hochrisiko-HPV von Niedrigrisiko-HPV unterschieden werden. Das erfindungsgemäße Verfahren umfasst eine Qualitätskontrolle von Pap-Abstrichen, welche beispielsweise Ausgangsmaterial für die Durchführung des Verfahrens liefern. Um aussagekräftige Ergebnisse liefern zu können und damit falsch-negative Diagnosen zu vermeiden, ist es zweckmäßig, mit dem Abstrich ausreichend Material zu entnehmen. Gelangt durch einen unzureichenden Abstrich kein oder zu wenig humanes Zellmaterial, insbesondere Zellen oder Zellfragmente, zur weiteren Analyse, können entsprechende Erreger nicht nachgewiesen werden, und es kommt zu falsch-negativen Diagnosen. Um dieses Risiko auszuschließen, ist es bei einer bevorzugten Ausführungsform vorgesehen, dass ein entsprechender Kontrollschritt im Testverfahren enthalten ist, so dass nur dann eine Aussage über die HPV-Infektion bzw. die entsprechenden Erreger getroffen wird, wenn ausreichend Zellmaterial vorgelegen hat.

Darüber hinaus umfasst das Verfahren gemäß der vorliegenden Erfindung zweckmäßigerweise Testschritte für HPV-Erreger des Niedrigrisiko-Typs und Testschritte für HPV-Erreger des Hochrisiko-Typs. Mit Hilfe der Tests für die HPV-Erreger beider Typen können zum einen Aussagen getroffen werden, ob überhaupt eine HPV-Infektion vörliegt. Zum anderen können Aussagen darüber gemacht werden, ob bei dem vorliegenden Typ des Erregers mit einer höheren oder mit einer niedrigeren Wahrscheinlichkeit damit zu rechnen ist, dass sich ein Karzinom, insbesondere ein Zervixkarzinom, entwickelt oder dass bereits ein solches vorliegt.

In den offenbarten Verfahren werden die Primer in Gruppen, vorzugsweise in Gruppen für gemeinsame Bereiche und für benachbarte Bereiche der DNA, insbesondere der HPV-DNA, eingesetzt.

Die Primer werden in Gruppen mit Forward- und Reverse-Primern eingesetzt, wobei die Forward- und Reverse-Primer degenerierte Nukleotideinheiten umfassen.

Überraschenderweise zeigt sich, dass der Einsatz von Gruppen von Forward- und/oder Reverse-Primern die Degeneration in DNA-Abschnitten, insbesondere in HPV-DNA-Abschnitten, vorteilhaft kompensiert und/oder eine erfolgreiche Amplifikation von DNA-Material, insbesondere von HPV-DNA-Material, begünstigt. Darüberhinaus ist hervorzuheben, dass durch eine Verteilung der Primer, insbesondere durch eine Verteilung mehrerer, vorzugsweise einander ergänzender, Paare von Forward- und Reverse-Primern über einen größeren DNA-Bereich, insbesondere einen HPV-DNA-Bereich, mit mehreren Zielsequenzen die Sicherheit des Nachweises erhöht wird. Somit wird die Wahrscheinlichkeit falsch-negativer Testergebnisse auf ein Minimum reduziert.

Es ist offenbart, dass mindestens ein Primer eingesetzt wird. Vorteilhaft wird ein Gemisch aus einem Forward-und einem Reverse-Primer eingesetzt, vorzugsweise ein Gemisch aus einer Gruppe von Forward-Primern und einer Gruppe von Reverse-Primern. Die Forward- und Reverse-Primer binden zweckmäßigerweise so an der HPV-DNA und werden vorteilhaft während der Amplifikation so verlängert, dass die resultierenden Amplifikate einen gemeinsamen HPV-DNA-Bereich aufweisen, insbesondere dass sich die resultierenden Amplifikate komplementär ergänzen. Zweckmäßigerweise wird zusätzlich mindestens ein weiterer Primer eingesetzt, vorteilhafterweise ein Gemisch aus mindestens einem Forward- und mindestens einem Reverse-Primer, so dass zu dem HPV-DNA-Bereich mindestens ein weiterer benachbarter Bereich amplifiziert wird.

Wie bereits beschrieben, ist es bevorzugt, die PCR oder PCR-ähnliche Methoden als Amplifikationsverfahren zu verwenden. Die PCR oder PCR-ähnliche Methoden werden in vitro durchgeführt und sind sehr sensitiv, so dass sehr geringe Mengen an Ausgangsmaterial amplifiziert werden können.

In einer weiteren bevorzugten Ausführungsform des Verfahrens werden die Oligonukleotidsonden in einer reversen Hybridisierung eingesetzt

Die reverse Hybridisierung hat den Vorteil, dass eine Probe gleichzeitig mit einer Vielzahl von Sonden mit vertretbarem Zeit- und Arbeitsaufwand untersucht werden kann. Dies erlaubt eine gleichzeitige Untersuchung mehrerer Zielsequenzen und gewährt damit die gleichzeitige Untersuchung mehrerer HPV-Typen. Die reverse Hybridisierung beruht auf einer Immobilisierung der Oligonukleotidsonden auf einem Träger und anschließender Hybridisierung der amplifizierten und markierten DNA aus der zu untersuchenden Probe. Hybride mit Fehlpaarungen werden zweckmäßigerweise in einem folgenden stringenten Waschschritt entfernt. An die Oligonukleotidsonden binden somit vorteilhafterweise dazu komplementäre Nukleotidsequenzen, die in der Probe enthalten waren. Durch die Markierung können die aus den Amplifikaten und den Sonden gebildeten Hybride erkannt werden. Somit lassen sich Rückschlüsse auf die in der Probe enthaltenen HPV-Typen ziehen. Als Trägermaterialien kommen beispielsweise Nylon, Nitrocellulose oder Polyvinylidendifluorid (PVDF) in Frage. Die Träger, können in Form von Membranen, insbesondere als Teststreifen, Mikrotiterplatten oder Objektträger (DNA-Chips) eingesetzt werden. Als Markierung können beispielsweise Fluo-reszenzfarbstoffe oder Haptene und/oder radioaktiv markierte Nukleotide dienen. Eine anschließende Detektion kann mit antikörpergebundenen Enzymen, sogenannten Konjugaten, in Verbindung mit einem chromogenen oder luminogenen Substrat erfolgen. Eine anschließende lichterzeugende Reaktion, insbesondere eine Farbreaktion, Chemolumineszenz oder Fluoreszenz, zeigt eine Bindung markierter Amplifikate und somit die Anwesenheit bestimmter Infektionserreger in dem untersuchten Material an, insbesondere die Anwesenheit von bestimmten HPV-Typen.

In einer weiteren bevorzugten Ausführungsform stammt die HPV-DNA aus Probenmaterialien von Biopsien oder Abstrichen, insbesondere Pap-Abstrichen. Vorzugsweise wird die HPV-DNA aus den Probenmaterialien isoliert und der PCR zugeführt. Allerdings sind auch DNA-Amplifikationen aus unaufbereiteten Proberimaterialien möglich. Auch mit solchen Ausgangsmaterialien ist im Rahmen der PCR die Amplifikation der HPV-DNA durchführbar.

In einer weiteren Ausführungsform weist das Verfahren erfindungsgemäß eine Qualitätsüberwachung der Nachweise durch mindestens einen Verfahrensschritt zur Kontrolle der Qualität der Probenmaterialien auf. Die Qualitätsüberwachung der Probenmaterialien wird gewöhnlich durch Nachweis von humaner DNA, vorzugsweise von Glycerinaldehyd-3-phosphat-dehydrogenase (GAPDH)-DNA, vorgenommen.

Voraussetzung für aussagekräftige Testergebnisse ist in der Regel, dass im Probenmaterial humane Zellen oder Zellfragmente enthalten sind. Erfindungsgemäß wird daher vorzugsweise getestet, ob humane DNA im Abstrichmaterial vorhanden ist. Auf diese Weise werden falsch-negative Diagnosen vermieden. Für derartige Nachweise kann die PCR mit geeigneten Primern in Frage kommen. Entstandene PCR-Produkte können vorteilhafterweise mit Hilfe von geeigneten Gensonden nachgewiesen werden. Besonders vorteilhaft ist der Nachweis der DNA von humanen Enzymen, insbesondere von konstitutiv synthetisierten Enzymen. Erfindungsgemäß ist es insbesondere vorgesehen, auf Glycerinaldehydphosphat-Dehydrogenase (GAPDH)-DNA zu testen. Selbstverständlich können auch andere humane DNA-Abschnitte für die Nachweise gewählt werden. Voraussetzung hierfür ist, dass die DNA spezifisch erkannt, insbesondere amplifiziert, werden kann. Andere Möglichkeiten zur Kontrolle des Probenmaterials sind beispielsweise der Nachweis von bestimmten humanen Proteinen oder der Nachweis von bestimmten enzymatischen Aktivitäten.

In einer weiteren Ausführungsform des Verfahrens sind Mittel enthalten, die zum Nachweis von Herpes simplex-Viren (HSV) geeignet sind. Hierbei ist der HSV II-Erreger von besonderem Interesse. Es ist beispielsweise bekannt, dass der Nachweis von HSV II und HPV-Hochrisiko-Typen einen Hinweis auf ein erhöhtes Risiko darstellt, ein Karzinom, insbesondere Zervixkarzinom zu entwickeln. Erfindungsgemäß ist es daher bevorzugt vorgesehen, neben der Qualitätskontrolle der Probenmaterialien und der Typisierung der HPV-Erreger zusätzlich zu testen, ob HSV II-Erreger vorhanden sind. Hierdurch kann die Risikogruppe der Patientinnen, welche mit erhöhter Wahrscheinlichkeit Karzinome, insbesondere Zervixkarzinome entwickeln werden, erheblich eingegrenzt werden. Durch den kombinierten Nachweis von HSV II und HPV-Hochrisiko-Typen können also wesentlich zuverlässigere Prognosen gestellt werden.

Darüber hinaus kann es vorteilhaft sein, dass ebenfalls getestet wird, ob HSV I-Erreger vorliegen. Bei dem Herpes simplex-Virus handelt es sich um einen sehr verbreiteten Virus, wobei hier insbesondere zwei Typen relevant sind. Lediglich beim zweiten Typ, HSV II, liegt in Kombination mit HPV-Hochrisiko-Typen ein erhöhtes Risiko vor, Krebs, insbesondere Gebärmutterhalskrebs, zu entwickeln. HSV I ist in Bezug auf Gebärmutterhalskrebs oder dessen Entwicklung als unproblematisch anzusehen. Der Nachweis von HSV I erfolgt daher bevorzugt als Negativkontrolle. Insbesondere handelt es sich um ein wichtiges Ausschlußkriterium, wenn bekannt ist, dass eine Herpes-Infektion vorliegt und lediglich HSV I nachgewiesen werden kann. Kann HSV II nicht nachgewiesen werden, kann aufgrund der Negativkontrolle mit Sicherheit ausgeschlossen werden, dass dieser besonders problematische HSV-Typ vorliegt. Folglich ist das Risiko bei einer Infektion mit HPV-Hochrisiko-Typen für die Entwicklung von Karzinomen, insbesondere von Zervixkarzinomen, niedriger einzustufen.

In einer Ausführungsform des Verfahrens sind zusätzlich Nachweismittel für Erreger von *Chlamydia trachomatis* vorgesehen. Die Infektionsrate mit Chlamydien ist bei jungen Frauen sehr hoch. Eine Chlamydieninfektion heilt aber oftmals ohne weitere Symptome wieder ab. Jedoch können Chlamydien sowohl bei Frauen als auch bei Männern Unfruchtbarkeit verursachen. Sie werden zusammen mit HPV und Herpes als sexuell übertragbare Krankheiten (sexual transmitted diseases - STD) bezeichnet. Diese drei Erkrankungen machen in den Industrieländern zusammen weit über 90 % aller STD aus. Es ist daher von besonderem Interesse, einen Nachweis von Chlamydien in Kombination mit Herpes und HPV durchzuführen. Mit dem Nachweis der verschiedenen Erreger kann ein geeigneter Test für ein STD-Screening bereitgestellt werden. Das Screening kann mit den Probenmaterialien, beispielsweise aus Abstrichen, insbesondere zervikalen Abstrichen, und mit einer Kontrolle bzw. zwei Kontrollen ohne großen Aufwand durchgeführt werden.

Als weitere Module für Extranachweise kommen vor allem der Nachweis von *Treponema pallidum* als Erreger der Syphillis und von *Neisseria gonorrhoe* als Erreger von Gonorrhoe (Tripper) in Betracht. Diese verschiedenen zusätzlichen Nachweise können in der oben beschriebenen Weise mit geeigneten Primern und Gensonden erfolgen. Es werden jedoch auch andere Nachweismethoden, beispielsweise über Antikörper oder Vergleichbares, von der Erfindung mit umfaßt.

In einer weiteren Ausführungsform des erfindungsgemäßen Verfahrens sind Nachweismittel vorgesehen, die einen weiteren Risikofaktor zur Entwicklung eines Zervixkarzinoms zeigen. Bei dieser Untersuchung von HPV-Infektionen wird auf p16^{INK4a} getestet. Die DNA für p16^{INK4a} liegt im Chromosomensatz der humanen Zellen auch unter normalen Bedingungen vor. Sie codiert ein Tumor Suppressor Protein, welches cyclinabhängige Kinasen (CDK), insbesondere die cyclinabhängigen Kinasen (CDK)-4 und (CDK)-6, hemmt. CDK-4 und CDK-6 sind in der Lage regulatorisch wirkende Proteine, beispielsweise das regulatorisch wirkende Retinoblastom Protein (pRB), durch Phosphorylierung zu inaktivieren. Unphosphoryliertes pRB hemmt die Expression von p16^{INK4a}. Somit stehen pRB und p^{16INK4a} in einer wechselseitigen Beziehung zueinander. p16^{INK4a} schützt pRB vor der Phosphorylierung und damit vor der Inaktivierung durch die CDK und pRB hemmt die Expression von p16^{INK4a}. Eine Integration des HPV in das Genom der humanen Zelle geht für gewöhnlich mit einer Zerstörung des viralen E2-Suppressorgens einher. Das E2-Suppressorgen kontrolliert die Expression der viralen Gene E6 und E7. Die Bildung von E6- und E7-Genprodukten wird somit induziert. Die E6- und E7-Genprodukte sind Onkogene und stellen wesentliche Faktoren für eine Karzinogenese dar, da sie regulatorische Proteine wie das Tumor Suppressor Protein p53 und/oder das pRB von Wirtszellen binden und dadurch inaktivieren. Die Inaktivierung von pRB durch virales E7 führt daher zu einer Überexpression von p16^{INK4a}. Da die CDK-4 und CDK-6 weiterhin den G1 "Checkpoint" im Zellzyklus regulieren, führt die erhöhte Expression von p16^{INK4a} zu einer Mißregulation der CDK und somit zu einer Mißregulation des Zellzyklus. Das Vorliegen der p16^{INK4a}-mRNA bzw. des translatierten Proteins belegt die Integration des Virus in das Genom von menschlichen Zellen und die Mißregulation des Zellzyklus. Ein solches Stadium ist als unmittelbare Vorstufe von Karzinomen zu betrachten. In einem solchen Fall besteht daher ein absolut akutes Risiko zur Entwicklung von Zervixkarzinomen oder es kann sich bereits ein solches entwickelt haben.

Der Nachweis von p16^{INK4a} kann über einen entsprechenden Proteinnachweis erfolgen. Dies kann beispielsweise mittels geeigneter Antikörper geschehen. Besonders bevorzugt ist es jedoch, wenn die mRNA für p₁₆^{INK4a} nachgewiesen wird. Sie ist ein direktes Maß für das gebildete p₁₆^{INK4a}-Protein. Vorzugsweise erfolgt der Nachweis der mRNA über geeignete PCR-Primer. Hierbei wird vorteilhafterweise ausgenutzt, dass die mRNA bei ihrer Bildung prozessiert wird. Beispielsweise werden durch sogenanntes Spleißen vorhandene Introns herausgeschnitten (Splicing). Vorzugsweise wird daher zum Nachweis von p16^{INK4a} eine Sequenz gewählt, die nur in ihrer gespleißten Form vorliegt. Auf diese Weise ist sichergestellt, dass die im Genom vorhandene untranskribierte DNA für p16^{INK4a} nicht amplifiziert wird. Vorzugsweise wird über ein Intron hinweg unter Einsatz geeigneter Primer eine Sequenz von zwei Exons amplifiziert.

Für den Nachweis von p16^{INK4a}, insbesondere für den Nachweis der mRNA, wird vorzugsweise eine Kontrolle durchgeführt, die sicherstellt, dass das Ergebnis dieses Nachweises aussagekräftig ist. Als Kontrolle eignet sich hierfür in besonderer Weise der Nachweis von konstitutiv produzierter humaner mRNA. Eine geeignete Kontrollsequenz sollte also immer in einer gewissen Menge produziert werden, so dass gewährleistet ist, dass in der Probe Zellmaterial vorhanden ist, in welchem die Transkriptionsvorgänge in regulärer Weise ablaufen. Sollte dies nicht der Fall sein, hat auch das Ergebnis, dass keine p16^{INK4a}-mRNA in der Probe vorhanden ist, keine Aussagekraft. Eine geeignete Kontrolle gewährleistet also, dass derartige falsch-negative Aussagen vermieden werden. Ein bevorzugtes Beispiel für eine geeignete Kontrolle ist der Nachweis von mRNA für β2-Mikroglobulin, welche immer entsprechend gespleißt und konstitutiv transkribiert wird. Erfindungsgemäß verhindert ein Einsatz von intronübergreifenden Primersequenzen eine Koamplifikation von genomischer DNA.

Die vorliegende Erfindung löst das ihr zugrunde liegende technische Problem auch durch die Bereitstellung eines Kits zur Diagnose von Infektionen, insbesondere zur Durchführung eines Verfahrens gemäß der vorliegenden Erfindung, umfassend mindestens ein Behältnis, das mindestens eine Oligonukleotidsonde der Sequenzen SEQ ID NO : 25-48 aufweist und ein PCR System nach einem der vorliegenden Ansprüche 1-4, als Nachweismittel für Niedrigrisiko-HPV und/oder Nachweismittel für Hochrisiko-HPV.

Der Kit ist bevorzugterweise so aufgebaut, dass für die verschiedenen beschriebenen Nachweise und Kontrollen geeignete Primer eingesetzt werden, die für eine Amplifizierung der gewünschten Nukleinsäuresequenzen durch PCR-Reaktionen geeignet sind. Durch die Auswahl der Primer kann bestimmt werden, welche Nachweise und Kontrollen durchgeführt werden sollen. Vorteilhafterweise liegen hierbei die Primer in Form von Gemischen verschiedener Primer vor, so dass durch Auswahl eines bestimmten Gemisches entschieden werden kann, welche Nachweise geführt werden.

In einer weiteren offenbarten Form des Kits umfassen die Gemische Gruppen von Primern mit mindestens einer der Nukleotidsequenzen SEQ ID Nr. 49 bis 56 und/oder SEQ ID Nr. 57 bis 64 aus beiliegendem Sequenzprotokoll.

Diese Maßnahme hat den Vorteil Primer für sämtliche Hochrisiko-HPV-Typen in einem Kit bereitzustellen und somit nachweisen zu können. Im Falle von unklaren Ergebnissen, beispielsweise im Falle von schwachen Farbreaktionen, erlauben mehrere Gemische von Primern für verschiedene HPV-DNA-Bereiche eine ergänzende Kontrolle von Ergebnissen und erhöhen somit die Sicherheit der Nachweise.

In einer weiteren Ausführungsform des Kits sind die Oligonukleotidsonden auf einem Träger, insbesondere auf Trägermaterialien, immobilisiert. Als Trägermaterialien kommen insbesondere Nitrocellulose, Nylon oder PVDF in Frage. Die Charakterisierung der Amplifikate erfolgt durch die Hybridisierung mit den fixierten Oligonukleotidsonden. Im Anschluss an die Hybridisierung gewährleisten stringente Waschschritte, dass die Bindung der Amplifikate mit den Oligonukleotidsonden nur dann erhalten bleibt, wenn die Sequenz der Sonde weitgehend (je nach Sequenz zu ca. 80 %), vorzugsweise zu 100 %, zu der Sequenz des Amplifikats komplementär ist. Die fixierten Oligonukleotidsonden lassen auch harsche Bedingungen für die Waschschritte zu. Somit ist sichergestellt, dass nur spezifisch gebundene Amplifikate nachgewiesen werden. Unspezifisch gebundene Materialien, die die Tests verfälschen könnten, werden hierdurch ausgeschlossen.

In einer weiteren offenbarten Form des Kits sind die Oligonukleotidsonden in Gruppen, insbesondere in Gruppen mit den Nukleotidsequenzen SEQ ID Nr. 1 bis 28 und/oder SEQ ID Nr. 29 bis 48 umfasst.

Diese Maßnahme hat, wie für die Primer bereits beschrieben, den Vorteil, Oligonukleotidsonden für sämtliche Hochrisiko-HPV-Typen in einem Kit bereitzustellen und somit nachweisen zu können. Im Falle von unklaren Ergebnissen, beispielsweise im Falle von schwachen Farbreaktionen, erlauben die Gruppen von Oligonukleotidsonden für verschiedene HPV-DNA-Bereiche eine ergänzende Kontrolle von Ergebnissen und erhöhen somit die Sicherheit der Nachweise.

in einer weiteren Form des Kits wird zusätzlich ein Nachweis für den Erreger *Chlamydia trachomatis* durchgeführt.

In einer weiteren Form kann zusätzlich oder alternativ dazu auf die Erreger *Treponema pallidum* und/oder *Neisseria gonorrhoe* getestet werden.

Weiterhin ist es bevorzugt, dass als Nachweismittel und/oder Kontrollmittel Gensonden in dem erfindungsgemäßen Kit enthalten sind, die spezifisch mit den nachzuweisenden Sequenzen wechselwirken können. In besonders vorteilhafter Weise sind diese Gensonden zum Nachweis von PCR-Produkten vorgesehen, die durch eine Amplifizierung mittels geeigneter Primer in der beschriebenen Weise eingesetzt werden können. In einer bevorzugten Ausführungsform sind diese Gensonden auf mindestens einem Träger fixiert, so dass die verschiedenen Nachweise ohne großen Aufwand durchgeführt werden können. Beispielsweise kann der Träger so ausgestaltet sein, dass es sich dabei um einen Teststreifen mit den entsprechend fixierten Gensonden handelt. Dieser Teststreifen kann beispielsweise mit dem PCR-Reaktionsgemisch benetzt werden, so dass die darin enthaltenen PCR-Produkte auf dem Teststreifen verteilt werden, beispielsweise durch Kapillarkräfte, und so zu den verschiedenen Gensonden gelangen und hier bei positiven Ergebnissen wechselwirken. Diese Wechselwirkungen werden vorzugsweise durch geeignete Methoden sichtbar gemacht. Dies kann beispielsweise durch eine Markierung der PCR-Produkte während der PCR-Reaktion geschehen, so dass bei Wechselwirkung eine Farbreaktion oder eine andere nachweisbare Reaktion an entsprechender Stelle des Trägers, insbesondere des Teststreifens, auftritt.

Als weitere Möglichkeit für den Nachweis bestimmter Erreger können PCR-Primer in Kombination mit einer Sequenzierung der PCR-Produkte eingesetzt werden.

In einer weiteren Ausführungsform der Erfindung stammen die Probenmaterialien aus Biopsien oder Abstrichen, insbesondere Zervikalabstrichen.

In einer bevorzugten Ausführungsform des erfindungsgemäßen Kits sind weiterhin Reagenzien zur Isolierung von DNA und/oder mRNA vorhanden. Hierbei handelt es sich beispielsweise um geeignete Puffer, Stammlösungen und/oder Enzyme. Die Reagenzien können selbstverständlich auch vom Anwender bereitgestellt werden.

In einer weiteren bevorzugten Ausführungsform sind im erfindungsgemäßen Kit Reagenzien zur Durchführung von PCR-Reaktionen enthalten. Hierbei kann es sich wiederum um geeignete Puffer, Stammlösungen und/oder Enzyme handeln. Weiterhin können auch geeignete Probengefäße und ähnliches im erfindungsgemäßen Kit enthalten sein.

Ein großer Vorteil des offenbarten Kits zur Diagnose von HPV-Infektionen ist, dass der Kit modular aufgebaut sein kann und so eine stufenweise Diagnose der Infektionen möglich ist. So kann beispielsweise eine Typisierung des HPV-Erregers und der Nachweis für HSV IIvorgenommen werden, ohne dass ein Nachweis von p16^{INK4a} erfolgt. Der modulare Aufbau kann insbesondere so verwirklicht sein, dass verschiedene Primergemische zum Nachweis verschiedener Erreger- bzw. Kontrollkömbinationen angeboten werden, wobei also jeweils ein bestimmtes Gemisch ausgewählt werden kann. Die Gensonden selbst können vorteilhafterweise immer in ihrer Gesamtheit auf einem Träger, beispielsweise auf einem Teststreifen, vorhanden sein. Nur bei Auswahl bestimmter geeigneter Primer wird dann eine Farbreaktion bzw. eine nachweisbare Reaktion auftreten können. Dies hat den Vorteil, dass immer der gleiche Träger eingesetzt werden kann. Der modulare Aufbau ist dann durch die verschiedenen Primergemische verwirklicht. Andererseits können auch unterschiedliche Träger mit verschiedenem Gensondenbesatz als modulare Komponenten bei immer gleicher Primerzusammensetzung eingesetzt werden. Hier sind alle Kombinationen möglich.

In einer weiteren Form weist der Kit Träger in Form von Teststreifen, Oligonukleotidsonden, insbesondere auf den Trägern aufgebrachte Oligonukleotidsonden, und Primer, insbesondere Forward- und/oder Reverse-Primer, in einem Set auf, insbesondere in einem Set, das auf die nachzuweisenden Zielsequenzen abgestimmt ist. Bevorzugterweise umfasst der offenbarte Kit sämtliche Nukleotidsequenzen SEQ ID Nr. 1 bis 64 aus beiliegendem Sequenzprotokoll.

Die verschiedenen Primer können jeweils in einem Reaktionsgefäß bereitgestellt werden, so dass auch die PCR-Reaktionen im Gemisch ablaufen. Vorteilhafterweise sollten die Primer für Reaktionen mit mRNA und für Reaktionen mit DNA voneinander getrennt sein. Es kann jedoch auch bevorzugt sein, dass sämtliche Reaktionen in einem Gefäß ablaufen oder dass die verschiedenen Reaktionen getrennt voneinander durchgeführt werden.

Die vorliegende Offenbarung betrifft weiterhin auch Teststreifen, umfassend Oligonukleotidsonden mit mindestens einer der Nukleotidsequenzen gemäß der vorliegenden Anmeldung, und auch die Verwendung von mindestens einer Oligonukleotidsonde gemäß der vorliegenden Anmeldung zur Herstellung von Teststreifen.

Ferner betrifft die vorliegende Offenbarung auch die Verwendung mindestens einer der offenbarten Nukleotidsequenzen, zum Nachweis von humanen Papillomaviren (HPV), insbesondere zur Diskriminierung zwischen Hochrisiko-HPV und Niedrigrisiko-HPV, und auch die Verwendung mindestens einer der Nukleotidsequenzen gemäß der vorliegenden Erfindung, zur Früherkennung von Karzinomerkrankungen, insbesondere von Präkanzerosen (kanzerogenen Vorstufen), vorzugsweise von Gebärmutterhalskrebs.

Somit eignen sich die offenbarten Primer- und oder Oligonukleotidsondensequenzen mit besonderem Vorteil zur Prävention von karzinogenen Erkrankungen.

Weitere Merkmale und Vorteile der Erfindung ergeben sich aus der nachfolgenden Beschreibung bevorzugter Ausführungsformen anhand von Beispielen in Verbindung mit den Unteransprüchen. Hierbei können die einzelnen Merkmale jeweils für sich allein oder zu mehreren in Kombination verwirklicht sein.

Die vorliegende Erfindung wird durch die folgenden Beispiele bevorzugter Ausführungsformen im Zusammenhang mit den folgenden Figuren und durch das beiliegende Sequenzprotokoll näher beschrieben und erläutert. Es zeigen:
- Fig. 1:: Darstellung eines Teststreifens mit verschiedenen Gensonden, und
- Fig. 2:: Darstellung von verschiedenen Ergebnissen für den Teststrei- fen gemäß der Fig. 1.

### Beispiel:

Das Beispiel verdeutlicht den Nachweis von Humanen Papillomaviren (HPV). In einem ersten Schritt wird eine PCR mit Zielsequenzen im E1-Genbereich durchgeführt. Anschließend erfolgt eine Charakterisierung erhaltener PCR-Produkte vorzugsweise mittels einer reversen Hybridisierung. Insbesondere werden Amplifikate an auf einem Träger fixierten Oligonukleotidsonden gebunden und nachgewiesen. Vorzugsweise besteht der Träger aus einer Membran, insbesondere einer Membran aus Nitrocellulose, vorzugsweise in Form eines Teststreifens. Eine Differenzierung in Hochrisiko-Typen und Niedrigrisiko-Typen lässt sich sowohl mittels Gruppen- als auch mittels Einzelnachweisen vornehmen.

### Material und Methoden:

Es werden zwei PCR Systeme verwendet. Das PCR-System 1 umfasst 4 PCR-Primersets A, B, C und D. Sequenzen der Primersets liegen alle im vorderen E1-Gen-Bereich von nt 850 bis 1750 im HPV-Genom (Position bezogen auf HPV16). Das PCR-System 2 umfasst die 4 PCR-Primersets E, F, G und H, deren Sequenzen alle im mittleren E1-Gen-Bereich von nt 1750 - 2000 liegen. Ein Primerset weist erfindungsgemäß ein Set aus Forward-Primern und ein Set aus Reverse-Primern auf. Nachweise lassen sich aber auch mit einzelnen Primer durchführen. Die Primer-Sets A und D bzw. E und H decken jeweils einen gemeinsamen Bereich, die Sets B und C bzw. F und G einen direkt angrenzenden Bereich ab. Die Sequenzen A-for, A-rev, B-for, B-rev, C-vor, C-rev, D-for und D-rev entsprechen den SEQ ID Nr. 49 bis 56 aus beiliegendem Sequenzprotokoll. Die Sequenzen E-for, E-rev, F-for, F-rev, G-for, G-rev, H-for und H-ref entsprechen den SEQ ID Nr. 57 bis 64 aus beiliegendem Sequenzprotokoll. Weitere Kombinationen aus Oligonukleotidsonden und/oder Primern sind möglich.

### PCR-System 1:

| | |
|---|---|
| Primersequenzen: | |
| A-for: | TAVAKGCWGTRYVKGYHSTAAAACGAAAGTWT |
| besonders: | |
| A-for1a: | AGAKGCWGTRCAGGTYCTAAAACGAAAGTAT |
| A-for1b: | TAAATGCTGTGTGTGCACTAAAACGAAAGTTT |
| A-for1c: | TACATGCTGTGTCTGCAGTAAAACGAAAGTTT |
| | |
| A-rev: | TTCCACTTCASWATTGCCATANCCGCTGTC |
| besonders: | |
| A-rev1a: | TTCCACTTCAGAATTGCCATAACCGCTGTC |
| A-rev1b: | TTCCACTTCAGTATTGCCATAYCCGCTGTC |
| A-rev1c: | TTCCACTTCACTATTGCCATAGCCGCTGTC |
| | |
| B-for: | CCAGAAGGTACMGAYGGGGADGGGWCGGG |
| besonders: | |
| B-for1a: | CCAGAAGGTACMGACGGGGAGGG |
| B-for1b: | GAAGGTACAGAYGGGGAWGGGWCGGG |
| B-rev: | TGTGCTGTCTCDHGCTCTGCCTGTWCACAA |
| besonders | |
| B-rev1a: | TGTGCTGTCTCTWGCTCTGCCTGTTCACAA |
| B-rev1b: | TGTGCTGTCTCRCGCTCTGCCTGTACACAA |
| | |
| C-for: | MYSTGAAGGTACAGAKGRKGAGGGG |
| besonders: | |
| C-for1a: | ACCTGAAGGTACAGAKGGGGAGGGG |
| C-for1b: | CTGTGAAGGTACAGAKGATGAGGGG |
| | |
| C-rev: | TMRBGGRCTRVCTADAWACTTTCGTTTTA |
| besonders: | |
| C-rev1a: | CGCGGRCTGMCTAGAAACTTTCGTTTTA |
| C-rev1b: | TAAKGGACTRSCTAWATACTTTCGTTTTA |
| | |
| D-for: | GMKRHHSTRYRSGMVYTAAAACGAAAGYWHWTAGG |
| besonders | |
| D-for1a: | GAGGCAGTACAGGCACTAAAACGAAAGCTATTAGG |
| D-for1b: | CTGTGCAGGACCTAAAACGAAAGTATTTAGG |
| D-for1c: | CTGACCTGTGCGAGTTAAAACGAAAGTACAT |
| D-for1d: | CTATAGTGCAGGAGTTAAAACGAAAGTACAT |
| D-rev: | TTCCACTTCAGWATWGCCATWKSYACTRTC |
| besonders | |
| D-rev1a: | CCACTTCAGTATTGCCATAKCCACTGTC |
| D-rev1b: | CCACTTCAGAATAGCCATAKCCACTGTC |
| D-rev1c: | TTCCACTTCAGTATTGCCATTGGTACTAT |

### Sonden:

Nachfolgend werden zum Einzelnachweis und/oder Gruppennachweis geeignete Oligonukleotidsonden beschrieben. Oligo 1 bis 24 entsprechen den SEQ ID Nr. 1 bis 24 aus beiliegendem Sequenzprotokoll. Oligo 1 bis 3 eignen sich zum Einzelnachweis für HPV 16, 18, und 45:

| | |
|---|---|
| Oligo 1: | TGATATTAGTGGATGTGTAGACAAT |
| Oligo 2: | TTTTATTGATACACAAGGAACATTT |
| Oligo 3: | TTTTATTGACACACAATTATCCATT |

Oligo 4 bis 7 eignen sich zum Gruppennachweis für HPV 31, 33, 35, und 39:

| | |
|---|---|
| Oligo 4: | AAGTGATATTAGTAGTTGTGTGGAT |
| Oligo 5: | AAGTGCTGCGGAGGACGTTGTTGAT |
| Oligo 6: | TAGCAGCGTGAGCTTATGTGTTAAT |
| Oligo 7: | CTTTATTGATGATTCCACAGATATT |

Oligo 8 bis 13 eignen sich zum Gruppennachweis für HPV 51, 52, 53, 56, 58 und 59:

| | |
|---|---|
| Oligo 8: | CTTTATAGATAGTGAAACTAGTATT |
| Oligo 9: | AAGTGCTGGGCAAGATGGTGTAGAA |
| Oligo 10: | GTTTATAGACAATAGTAATATAATA |
| Oligo 11: | GATTTATAGACGATTCATATATACA |
| Oligo 12: | AAGTGCTGTAGAGGACTGTGTGGAC |
| Oligo 13: | TTTTATTGATGATACCACAACAATT |

Oligo 14 bis 17 eignen sich zum Gruppennachweis für HPV 66, 68, 73, und 82:

| | |
|---|---|
| Oligo 14: | ATTTATAGACAATACACTTATAAAC |
| Oligo 15: | TTTTATTGATGATTCTACACATATTT |
| Oligo 16: | TGAAAAGAGATGAATTCATAGACA |
| Oligo 17: | TTTTATAGATACAAGTAATAGTATT |

Oligo 18 bis 24 eignen sich zum Gruppennachweis für HPV 6, 11, 40, 41, 42, 43 und 44:

| | |
|---|---|
| Oligo 18: | AAACACTATAGCCGAGGCAGTGGAA |
| Oligo 19: | AAGCAATGTAGCTAATGCAGTAGAA |
| Oligo 20: | ACAATACTCAGAACCGTCTATAGAC |
| Oligo 21: | CAGTGGCATAGACAGCACCACAGTG |
| Oligo 22: | CAGTGATTCACAGCACAGCATAGAC |
| Oligo 23: | ACACTGTTTACAGGAATCTGTAGAC |
| Oligo 24: | AAGTAATATTGAGCAGGCAGTGGAG |

### PCR-System 2 :

### Primersequenzen:

| | |
|---|---|
| E-for: | RAYWAACWGTDTTACARCATAGYTTT |
| besonders: | |
| E-for1: | ACAAACAGTITTACARCATAGYTTT |
| | |
| E-rev: | CATAWTWATAKGCWATDTCACTATC |
| besonders: | |
| E-rev1: | CATATTTATATGCWATITCACTATC |
| E-rev2: | CATAATAATATGCAATGTCACTATC |
| F-for: | MGMACAGGWATRTCMAATATTAGTG |
| besonders: | |
| F-for1: | GAACAGGWATRTCMAATATTAGTG |
| | |
| F-rev: | AWTBAWATGCHATRTCRCTTTCATCWGT |
| besonders: | |
| F-rev1: | AATTAAATGCTATGTCACTTTCATCAGT |
| F-rev2: | TTCAAATGCMATATCRCTTTCATCTGT |
| | |
| G-for: | RTATGGVRRNACACCWGAATGGAT |
| besonders: | |
| G-for1: | TATGGAGASACACCWGAATGGAT |
| G-for2: | TATGGSGAAACACCWGAATGGAT |
| G-for3: | TATGGGAGTACACCWGAATGGAT |
| | |
| G-rev: | WGCATTRCTRTCTRYRTCWGCYARYTGTGCATADW |
| besonders | |
| G-rev1: | GCATTGCTATCTATGTCTGCTAGTTGTGC |
| G-rev2: | GCATTGCTATCTGTATCAGCCAATTGTGC |
| G-rev3: | GCATTACTGTCTAYATCTGCTAAYTGTGC |
| | |
| H-for: | GGVYDWCMWRYRVRTGGGGHATGGTV |
| besonders: | |
| H-for1: | GGCTTACAAATGCATGGGGAATGGTC |
| H-for2: | GGGTGACCTGTAGATGGGGAATGGTA |
| H-for3: | GGACGTCATGCAAATGGGGTATGGT |
| H-for4: | GGCTAACCTGTACGTGGGGCATGGT |
| H-rev: | YKHDBWATYMWYTCYGGYRYBKYBCC |
| besonders: | |
| H-rev1: | CGTGTTATCCATTCTGGTGCTTCCCC |
| H-rev2: | CGCGTTATCCATTCCGGCGCCTCCCC |
| H-rev3: | TTTACAATTATTTCTGGCATTGCGCC |
| H-rev4: | TTAGCTATCCATTCYGGTGTTGTGCC |
| H-rev5: | CTTGTTATCCATTCCGGTGTTTCTCC |
| H-rev6: | CTTTGAATCCACTCTGGTGTGTCTCC |

### Sonden:

Nachfolgend werden zum Einzelnachweis und/oder Gruppennachweis geeignete Oligonukleotidsonden beschrieben.

Oligo 25 bis 48 entsprechen den SEQ ID Nr. 25 bis 48 aus beiliegendem Sequenzprotokoll. Oligo 25 bis 27 eignen sich zum Einzelnachweis für HPV 16, 18, und 45:

| | |
|---|---|
| Oligo 25: | AATGATTGTACATTTGAATTATCACAG |
| Oligo 26: | AGTAATGGGAGACACACCTGAGTGGA |
| Oligo 27: | AGTAAGTGGAGACACACCTGAGTGGA |

Oligo 28 bis 31 eignen sich zum Gruppennachweis für HPV 31, 33, 35, und 39:

| | |
|---|---|
| Oligo 28: | AATGACACAACATTTGATTTGTCCCAA |
| Oligo 29: | AATGATAATATATTTGATTTAAGTGAA |
| Oligo 30: | AATGATGCAATATTTGACCTATCTGAA |
| Oligo 31: | GGTAACAGGGGATACGCCAGAATGGA |

Oligo 32 bis 37 eignen sich zum Gruppennachweis für HPV 51, 52, 53, 56, 58 und 59:

| | |
|---|---|
| Oligo 32: | ACAACATAGTTTTGAGGATAGTACCT |
| Oligo 33: | GACAATAGCATATTCGATTTTGGAGAA |
| Oligo 34: | ACAACATAGCTTTGAGGACTGTCAAT |
| Oligo 35: | GCAACACAGTTTACAGGATAGTCAAT |
| Oligo 36: | AATGATGATATATTTGATTTAAGTGAA |
| Oligo 37: | AGTTATAGGGGAAACGCCCGAATGGA |

Oligo 38 bis 41 eignen sich zum Gruppennachweis für HPV 66, 68, 73, und 82:

| | |
|---|---|
| Oligo 38: | GCAACACAGTTTACAAGACAATCAAT |
| Oligo 39: | GGTGTGTGGCGACACGCCGGAATGGA |
| Oligo 40: | GATGATAGTCAATTTGACCTATCTCAA |
| Oligo 41: | ACAGCACAGTTTTGATGATAGCACGT |

Oligo 42 bis 48 eignen sich zum Gruppennachweis für HPV 6, 11, 40, 41, 42, 43 und 44:

| | |
|---|---|
| Oligo 42: | CGTTGCACGTACACTTGCAACGCTAT |
| Oligo 43: | CGTGGCACGTACATTAGGTACGTTAT |
| Oligo 44: | AGTTGTTAGACAGCTATCCAAAATGT |
| Oligo 45: | AGTGTACAATTGGTTCACAGCCAATT |
| Oligo 46: | AGTGTCCAAAGGCCTTAGTAAATTAT |
| Oligo 47: | AATAGTAAGACAGCTAGCACAAATGT |
| Oligo 48: | AGTGGCACGTATGATGGCAACCCGTT |

Die PCR erfolgt in Multiplex Ansätzen mit den Primersets A bis H in geeigneten Kombinationen. Vorzugsweise werden A und D bzw. E und H zusammen in einem Mix sowie die Primersets B und C bzw. F und G zusammen in einem Mix eingesetzt. Die PCR erfolgt nach einem geeigneten Temperaturprogramm, vorzugsweise mit
- 5 Minuten Denaturieren bei 95°C, 1 Zyklus
- 45 Sekunden Denaturieren bei 95°C, 30 Zyklen
- 45 Sekunden Annealing bei 40°C,
- 30 Sekunden Elongation bei 72°C,
- 8 Minuten Elongation bei 72°C. 1 Zyklus

### Probenaufarbeitung:

Abstrich- beziehungsweise Biopsiematerial wird in einem geeigneten kommerziell erhältlichen Transportmedium, in dem DNA und RNA intakt bleiben, angeliefert. Die DNA beziehungsweise RNA-Extraktion (für p16^{INK4a}) erfolgt über allgemein übliche Säulenreinigungsverfahren. Die gewonnene DNA beziehungsweise RNA kann in die PCR mit den jeweiligen Mixen der verschiedenen Primer modulartig eingesetzt werden.

### Testdurchführung:

1. Der Hybridisierungspuffer und der Stringent Wash Puffer werden auf 47°C vorgewärmt, alle anderen Lösungen auf Raumtemperatur angewärmt. Inkubationswannen entsprechend der Anzahl der Proben und der Kontrollen werden vorbereitet. Vertiefungen, die benutzt werden sollen, werden am Rand markiert. Bereits gebrauchte Kavitäten werden nicht noch einmal verwenden.
2. In die markierten Kavitäten werden je 40 µl Denaturierungsreagenz pipettiert.
3. Je 20 µl Amplifikat von jedem der zu untersuchenden PCR-Ansätze werden zu dem Tropfen Denaturierungsreagenz pipettiert, gut gemischt und 5 Minuten bei Raumtemperatur inkubiert.
4. Es werden jeweils vorsichtig 1 ml vorgewärmte und gemischte Hybridisierungspuffer hinzugeben.
5. Die einzelnen Streifen werden mit einer Pinzette aus dem Röhrchen entnommen und in die Inkubationswanne gelegt. Die Streifen müssen vollständig mit Flüssigkeit bedeckt sein und die beschichtete Seite soll nach oben weisen. Dies gilt auch für alle nachfolgenden Inkubations-und Waschschritte. Streifen, die sich z. B. durch Zugabe von Waschpuffer wenden, werden mit einer Pinzette an einem Ende aufgenommen und zurückgedreht.
6. Die Wanne wird für 30 min. bei 47°C im Schüttelwasserbad inkubiert (Frequenz beachten).
7. Hybridisierungspuffer wird vollständig abgegossen und die Streifen anschließend zweimal 1 Minute mit je 1 ml vorgewärmter und gemischter Stringent-Waschlösung bei Raumtemperatur auf dem Horizontalschüttler (Frequenz!) gewaschen.
8. Jeweils 1 ml vorgewärmte Stringent-Waschlösung wird zugegeben und 15 Minuten bei 47°C im Wasserbad unter Schütteln inkubiert.
9. Von diesem Schritt an wird bei Raumtemperatur gearbeitet. Die Lösung wird abgeklopft und die Streifen zweimal mit je 1 ml Waschlösung je 1 Minute gewaschen (Schüttler).
10. 1 ml vorbereitetes Konjugat (Konjugat-Konzentrat im Verhältnis 1:100 mit Konjugat-Puffer verdünnt) wird zu jedem Streifen gegeben und 30 Minuten auf dem Horizontalschüttler inkubiert.
11. Das Konjugat wird entfernt und dreimal je 1 Minute mit je 1 ml Waschlösung gewaschen (Horizontalschüttler).
12. Je 1 ml auf Raumtemperatur erwärmtes Substrat wird in die Kavitäten pipettiert und, je nachdem wie rasch die Farbreaktion abläuft, zwischen 10 und 20 Minuten auf dem Horizontalschüttler inkubiert.
13. Die Reaktion wird durch zweimaliges Waschen mit destilliertem Wasser gestoppt. Die Streifen werden mit einer Pinzette aus den Kavitäten entnommen und zum Trocknen auf Filterpapier gelegt.

Die getrockneten Streifen werden anschließend ausgewertet und lichtgeschützt aufbewahrt.

### Zuordnung der HPV-Primer:

Mittels der Primer erfolgt noch keine Differenzierung nach HPV-Typen. Die Primer gewährleisten jedoch, dass möglichst alle relevanten HPV-Genotypen amplifiziert werden. Durch die reverse Hybridisierung erfolgt die Differenzierung in Hochrisiko ("high-risk")- und Niedrigrisiko ("low-risk")-HPV-Typen.

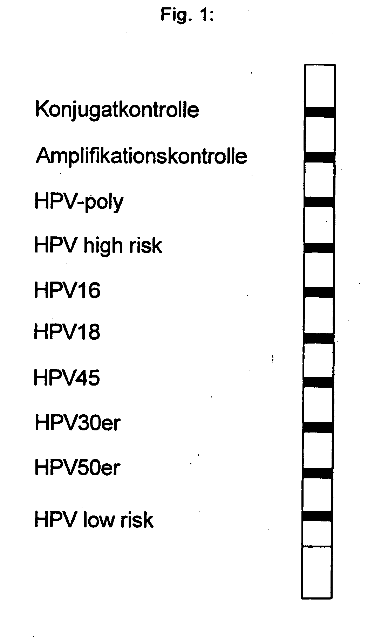

Figur 1 zeigt ein Beispiel für einen möglichen Aufbau eines Teststreifens gemäß der vorliegenden Erfindung mit entsprechenden Gensonden, auf dem die aufgearbeiteten Proben aufgetragen werden können, so dass diese mit Sequenzen der Gensonden interagieren. Ohne darauf beschränkt zu sein, können sowohl das beschriebene PCR-System 1 als auch das PCR-System 2 eingesetzt werden. Im Weiteren ist beispielhaft das PCR-System 1 beschrieben. Für das PCR-System 2 sind die Erläuterungen auf dessen Oligonukleotidsonden zu übertragen.

In Fig. 1 sind insgesamt 9 Testbereiche, auch als Reaktionszonen bezeichnet, definiert und entwicklungsfähig. Eine Konjugatkontrolle dokumentiert die Effizienz der Konjugatbindung. Sie muss zweckmäßigerweise immer entwickelt sein. Eine Amplifikationskontrolle muss ebenfalls immer entwickelt sein. Sie weist eine Sonde für ein sogenanntes "housekeeping" Gen auf, das in jeder humanen DNA-Probe zu finden ist. Im vorliegenden Fall handelt es sich bei dem "housekeeping" Gen um Glycerinaldehydphosphat-Dehydrogenase (GAPDH). Somit dient die Reaktionszone als Kontrolle von DNA-Isolierung, DNA-Amplifikation und Hybridisierung. Sind diese Reaktionszonen nicht entwickelt, liegt eine falsch negative Reaktion vor. In diesem Fall muß der Test wiederholt werden.

Weiterhin sind in der Reaktionszone HPV-poly alle Oligo 1 bis 24 für alle nachzuweisenden HPV des PCR-Systems 1 aufgebracht. Diese Reaktionszone ist entwickelt, wenn die DNA eines oder mehrerer der angeführten HPV-Genotypen im Probenmaterial enthalten sind. HPV high risk enthält alle Oligo 1 1 bis 17. Demgemäß bedeutet eine positive Reaktion, dass das untersuchte Probenmaterial einen oder mehrere HPV der Hochrisiko-Typen HPV16, 18, 31, 33, 35, 39, 45, 51, 52, 53, 56, 58, 59, 66, 68, 73, 82 enthält. HPV 16 enthält Oligo 1, HPV 18 enthält Oligo 2, HPV 45 enthält Oligo 3. Diese drei Reaktionszonen ermöglichen zusätzlich Einzelnachweise dieser drei häufig vorkommenden Hochrisiko-Genotypen. HPV 30er enthält alle Oligo 4 bis 7 und ermöglicht den Nachweis von Stämmen der Hochrisiko-Typen HPV31, 33, 35 und 39. HPV 50er enthält alle Oligo 8 bis 13. Diese Reaktionszone kann anzeigen, ob die Hochrisiko-Typen 51, 52, 53, 56 58 oder 59 vorhanden sind. HPV low risk enthält alle Oligo 18 bis 24 und steht für die Niedrigrisiko-Typen HPV6, 11, 40, 42, 43 und 44. Dieses Muster kann in Kombination mit weiteren Banden auf dem Teststreifen verwendet werden, um fakultativ weitere Erreger nachweisen zu können. Gegebenenfalls kann auch der Einsatz verkürzter Teststreifen, auf denen bestimmte Reaktionszonen fehlen, sinnvoll sein.

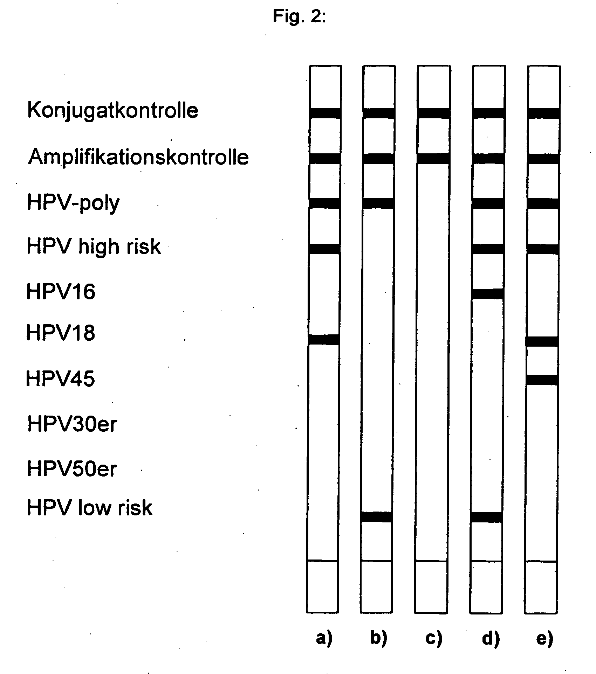

Fig. 2 zeigt mögliche Testergebnisse für Teststreifen der Fig. 1. In Fig. 2a) wird ein mögliches Ergebnis gezeigt, bei dem eine Infektion mit Hochrisiko-HPV-Typen vorliegt. HPV18 konnte nachgewiesen werden. Fig. 2b) zeigt ein mögliches positives Testergebnis in der Reaktionszone HPV-poly bei einer gleichzeitigen positiven Reakton mit Niedrigrisiko-HPV-Typen. Solch ein Testergebnis würde auf ein geringes Risiko für ein Zervixkarzinom oder dessen Entwicklung schließen lassen. Fig. 2c) zeigt ein negatives Testergebnis. Keiner der nachweisbaren HPV-Genotypen ist vorhanden. Fig. 2d) zeigt ein mögliches Testergebnis bei einer gleichzeitigen Hochrisiko-HPV- und Niedrigrisiko-HPV-Infektion. Fig. 2e) zeigt an, das Hochrisiko-HPV-Typen vorhanden sind, daneben wurden HPV18 und HPV45 nachgewiesen. In allen Testergebnissen würde eine nur schwach entwickelte GAPDH Kontrolle auf zu wenig DNA in dem Probenmaterial schließen lassen. Solch ein Testergebnis würde nicht ausgewertet werden, da das Probenmaterial offensichtlich zu wenige Zellen und/oder zu wenig PCR-Hemmstoff enthielt.

### Sequenzprotokoll

<110> A.I.D. Autoimmun Diagnostika GmbH
<120> Papillomaviren Nachweis
<130> P 46 349 DE
<160> 64
<170> Patentln version 3.3
<210> 1
   <211> 25
   <212> DNA
   <213> Human papillomavirus
<400> 1
   tgatattagt ggatgtgtag acaat 25
<210> 2
   <211> 25
   <212> DNA
   <213> Human papillomavirus
<400> 2
   ttttattgat acacaaggaa cattt 25
<210> 3
   <211> 25
   <212> DNA
   <213> Human papillomavirus
<400> 3
   ttttattgac acacaattat ccatt 25
<210> 4
   <211> 25
   <212> DNA
   <213> Human papillomavirus
<400> 4
   aagtgatatt agtagttgtg tggat 25
<210> 5
   <211> 25
   <212> DNA
   <213> Human papillomavirus
<400> 5
   aagtgctgcg gaggacgttg ttgat 25
<210> 6
   <211> 25
   <212> DNA
   <213> Human papillomavirus
<400> 6
   tagcagcgtg agcttatgtg ttaat 25
<210> 7
   <211> 25
   <212> DNA
   <213> Human papillomavirus
<400> 7
   ctttattgat gattccacag atatt 25
<210> 8
   <211> 25
   <212> DNA
   <213> Human papillomavirus
<400> 8
   ctttatagat agtgaaacta gtatt 25
<210> 9
   <211> 25
   <212> DNA
   <213> Human papillomavirus
<400> 9
   aagtgctggg caagatggtg tagaa 25
<210> 10
   <211> 25
   <212> DNA
   <213> Human papillomavirus
<400> 10
   gtttatagac aatagtaata taata 25
<210>. 11
   <211> 25
   <212> DNA
   <213> Human papillomavirus
<400> 11
   gatttataga cgattcatat ataca 25
<210> 12
   <211> 25
   <212> DNA
   <213> Human papillomavirus
<400> 12
   aagtgctgta gaggactgtg tggac 25
<210> 13
   <211> 25
   <212> DNA
   <213> Human papillomavirus
<400> 13
   ttttattgat gataccacaa caatt 25
<210> 14
   <211> 25
   <212> DNA
   <213> Human papillomavirus
<400> 14
   atttatagac aatacactta taaac 25
<210> 15
   <211> 26
   <212> DNA
   <213> Human papillomavirus
<400> 15
   ttttattgat gattctacac atattt 26
<210> 16
   <211> 24
   <212> DNA
   <213> Human papillomavirus
<400> 16
   tgaaaagaga tgaattcata gaca 24
<210> 17
   <211> 25
   <212> DNA
   <213> Human papillomavirus
<400> 17
   ttttatagat acaagtaata gtatt 25
<210> 18
   <211> 25
   <212> DNA
   <213>Human papillomavirus
<400> 18
   aaacactata gccgaggcag tggaa 25
<210> 19
   <211> 25
   <212> DNA
   <213> Human papillomavirus
<400> 19
   aagcaatgta gctaatgcag tagaa 25
<210> 20
   <211> 25
   <212> DNA
   <213> Human papillomavirus
<400> 20
   acaatactca gaaccgtcta tagac 25
<210> 21
   <211> 25
   <212> DNA
   <213> Human papillomavirus
<400> 21
   cagtggcata gacagcacca cagtg 25
<210> 22
   <211> 25
   <212> DNA
   <213> Human papillomavirus
<400> 22
   cagtgattca cagcacagca tagac 25
<210> 23
   <211> 25
   <212> DNA
   <213> Human papillomavirus
<400> 23
   acactgttta caggaatctg tagac 25
<210> 24
   <211> 25
   <212> DNA
   <213> Human papillomavirus
<400> 24
   aagtaatatt gagcaggcag tggag 25
<210> 25
   <211> 27
   <212> DNA
   <213> Human papillomavirus
<400> 25
   aatgattgta catttgaatt atcacag 27
<210> 26
   <211> 26
   <212> DNA
   <213> Human papillomavirus
<400> 26
   agtaatggga gacacacctg agtgga 26
<210> 27
   <211> 26
   <212> DNA
   <213> Human papillomavirus
<400> 27
   agtaagtgga gacacacctg agtgga 26
<210> 28
   <211> 27
   <212> DNA
   <213> Human papillomavirus
<400> 28
   aatgacacaa catttgattt gtcccaa 27
<210> 29
   <211>27
   <212> DNA
   <213> Human papillomavirus
<400> 29
   aatgataata tatttgattt aagtgaa 27
<210> 30
   <211> 27
   <212> DNA
   <213> Human papillomavirus
<400> 30
   aatgatgcaa tatttgacct atctgaa 27
<210> 31
   <211> 26
   <212> DNA
   <213> Human papillomavirus
<400> 31
   ggtaacaggg gatacgccag aatgga 26
<210> 32
   <211> 26
   <212> DNA
   <213> Human papillomavirus
<400> 32
   acaacatagt tttgaggata gtacct 26
<210> 33
   <211> 27
   <212> DNA
   <213> Human papillomavirus
<400> 33
   gacaatagca tattcgattt tggagaa 27
<210> 34
   <211> 26
   <212> DNA
   <213> Human papillomavirus
<400> 34
   acaacatagc tttgaggact gtcaat 26
<210> 35
   <211> 26
   <212> DNA
   <213> Human papillomavirus
<400> 35
   gcaacacagt ttacaggata gtcaat 26
<210> 36
   <211> 27
   <212> DNA
   <213> Human papillomavirus
<400> 36
   aatgatgata tatttgattt aagtgaa 27
<210> 37
   <211> 26
   <212> DNA
   <213> Human papillomavirus
<400> 37
   agttataggg gaaacgcccg aatgga 26
<210> 38
   <211> 26
   <212> DNA
   <213> Human papillomavirus
<400> 38
   gcaacacagt ttacaagaca atcaat 26
<210> 39
   <211> 26
   <212> DNA
   <213> Human papillomavirus
<400> 39
   ggtgtgtggc gacacgccgg aatgga 26
<210> 40
   <211> 27
   <212> DNA
   <213> Human papillomavirus
<400> 40
   gatgatagtc aatttgacct atctcaa 27
<210> 41
   <211> 26
   <212> DNA
   <213> Human papillomavirus
<400> 41
   acagcacagt tttgatgata gcacgt 26
<210> 42
   <211> 26
   <212> DNA
   <213> Human papillomavirus
<400> 42
   cgttgcacgt acacttgcaa cgctat 26
<210> 43
   <211> 26
   <212> DNA
   <213> Human papillomavirus
<400> 43
   cgtggcacgt acattaggta cgttat 26
<210> 44
   <211> 26
   <212> DNA
   <213> Human papillomavirus
<400> 44
   agttgttaga cagctatcca aaatgt 26
<210> 45
   <211> 26
   <212> DNA
   <213> Human papillomavirus
<400> 45
   agtgtacaat tggttcacag ccaatt 26
<210> 46
   <211> 26
   <212> DNA
   <213> Human papillomavirus
<400> 46
   agtgtccaaa ggccttagta aattat 26
<210> 47
   <211> 26
   <212> DNA
   <213> Human papillomavirus
<400> 47
   aatagtaaga cagctagcac aaatgt 26
<210> 48
   <211> 26
   <212> DNA
   <213> Human papillomavirus
<400> 48
   agtggcacgt atgatggcaa cccgtt 26
<210> 49
   <211> 32
   <212> DNA
   <213> Human papillomavirus
<400> 49
   tavakgcwgt ryvkgyhsta aaacgaaagt wt 32
<210> 50
   <211> 30
   <212> DNA
   <213> Human papillomavirus
<220>
   <221> misc-feature
   <222> (22)..(22)
   <223> n is g or a or t or c
<400> 50
   ttccacttca swattgccat anccgctgtc 30
<210> 51
   <211> 29
   <212> DNA
   <213> Human papillomavirus
<400> 51
   ccagaaggta cmgaygggga dgggwcggg 29
<210> 52
   <211> 30
   <212> DNA
   <213> Human papillomavirus
<400> 52
   tgtgctgtct cdhgctctgc ctgtwcacaa 30
<210> 53
   <211> 25
   <212> DNA
   <213> Human papillomavirus
<400> 53
   mystgaaggt acagakgrkg agggg 25
<210> 54
   <211> 29
   <212> DNA
   <213> Human papillomavirus
<400> 54
   tmrbggrctr vctadawact ttcgtttta 29
<210> 55
   <211> 35
   <212> DNA
   <213> Human papillomavirus
<400> 55
   gmkrhhstry rsgmvytaaa acgaaagywh wtagg 35
<210> 56
   <211> 30
   <212> DNA
   <213> Human papillomavirus
<400> 56
   ttccacttca gwatwgccat wksyactrtc 30
<210> 57
   <211> 26
   <212> DNA
   <213> Human papillomavirus
<400> 57
   raywaacwgt dttacarcat agyttt 26
<210> 58
   <211> 25
   <212> DNA
   <213> Human papillomavirus
<400> 58
   catawtwata kgcwatdtca ctatc 25
<210> 59
   <211> 25
   <212> DNA
   <213> Human papillomavirus
<400> 59
   mgmacaggwa trtcmaatat tagtg 25
<210> 60
   <211> 28
   <212> DNA
   <213> Human papillomavirus
<400> 60
   awtbawatgc hatrtcrctt tcatcwgt 28
<210> 61
   <211> 24
   <212> DNA
   <213> Human papillomavirus
<220>
   <221> misc-feature
   <222> (10)..(10)
   <223> n is g or a or t or c
<400> 61
   rtatggvrrn acaccwgaat ggat 24
<210> 62
   <211> 35
   <212> DNA
   <213> Human papillomavirus
<400> 62
   wgcattrctr tctryrtcwg cyarytgtgc atadw 35
<210> 63
   <211> 26
   <212> DNA
   <213> Human papillomavirus
<400> 63
   ggvydwcmwr yrvrtggggh atggtv 26
<210> 64
   <211> 26
   <212> DNA
   <213> Human papillomavirus
<400> 64
   ykhdbwatym wytcyggyry bkybcc 26

## Patentansprüche

1. PCR-System, umfassend ein Primer-Set aus einem Forward-Primer mit der Nukleotidsequenz SEQ ID Nr. 57 und einem Reverse-Primer mit der Nukleotidsequenz SEQ ID Nr. 58, ein Primer-Set aus einem Forward-Primer mit der Nukleotidsequenz SEQ ID Nr. 59 und einem Reverse-Primer mit der Nukleotidsequenz SEQ ID Nr. 60, einem Primer-Set aus einem Forward-Primer mit der Nukleotidsequenz SEQ ID Nr. 61 und einem Reverse-Primer mit der Nukleotidsequenz SEQ ID Nr. 62 und einem Primer-Set aus einem Forward-Primer mit der Nukleotidsequenz SEQ ID Nr. 63 und einem Reverse-Primer mit der Nukleotidsequenz SEQ ID Nr. 64.

2. PCR-System nach Anspruch 1, **dadurch gekennzeichnet, dass** Primer markiert sind, insbesondere mit Biotin.

3. PCR-System nach Anspruch 1 oder 2 zur Anwendung beim Nachweis von humanen Papillomaviren (HPV), insbesondere zur Diskriminierung zwischen Hochrisiko-Typen und Niedrigrisiko-Typen von humanen Papillomaviren (HPV).

4. PCR-System nach Anspruch 1 oder 2 zur Anwendung bei der Früherkennung von Karzinomerkrankungen, insbesondere von Präkanzerosen davon, vorzugsweise von Gebärmutterhalskrebs.

5. Verfahren zur Diagnose von Infektionen mit humanen Papillomaviren (HPV), das folgende Verfahrensschritte umfasst,
- Nachweis von DNA mit
a) Kontrollieren der Qualität von Proben
b) Testen auf Niedrigrisiko-HPV
c) Testen auf Hochrisiko-HPV
**dadurch gekennzeichnet, dass** der DNA-Nachweis über spezifische Bereiche von HPV-DNA unter Einsatz von Oligonukleotidsonden mit mindestens einer der Nukleotidsequenzen SEQ ID Nr. 25 bis 48 und einem PCR-System nach einem der Ansprüche 1 bis 4 vorgenommen wird.

6. Verfahren nach Anspruch 5, **dadurch gekennzeichnet, dass** die Primer in Gruppen, vorzugsweise in Gruppen für gemeinsame Bereiche und/ oder für benachbarte Bereiche der DNA, insbesondere der HPV-DNA, eingesetzt werden.

7. Verfahren nach Anspruch 5 oder 6, **dadurch gekennzeichnet, dass** die PCR oder PCR-ähnliche Methoden als Amplifikationsverfahren verwendet werden.

8. Verfahren nach einem der Ansprüche 5 bis 7, **dadurch gekennzeichnet, dass** die Oligonukleotidsonden in einer reversen Hybridisierung eingesetzt werden.

9. Kit zur Diagnose von Infektionen mit HPV, insbesondere zur Durchführung eines Verfahrens gemäß einem der Ansprüche 5 bis 8, umfassend mindestens ein Behältnis, das mindestens eine Oligonukleotidsonde mit mindestens einer der Nukleotidsequenzen SEQ ID Nr. 25 bis 48 aufweist, und ein PCR-System nach einem der Ansprüche 1 bis 4 als Nachweismittel für Niedrigrisiko-HPV und/oder Nachweismittel für Hochrisiko-HPV.

10. Kit nach Anspruch 9, weiter **dadurch gekennzeichnet, dass** die mindestens eine Oligonukleotidsonde auf einem Träger, insbesondere auf einem Teststreifen, immobilisiert ist.

## Claims

1. PCR system comprising a primer set consisting of a forward primer having the nucleotide sequence SEQ ID No. 57 and a reverse primer having the nucleotide sequence SEQ ID No. 58, a primer set consisting of a forward primer having the nucleotide sequence SEQ ID No. 59 and a reverse primer having the nucleotide sequence SEQ ID No. 60, a primer set consisting of a forward primer having the nucleotide sequence SEQ ID No. 61 and a reverse primer having the nucleotide sequence SEQ ID No. 62, and a primer set consisting of a forward primer having the nucleotide sequence SEQ ID No. 63 and a reverse primer having the nucleotide sequence SEQ ID No. 64.

2. PCR system according to Claim 1, **characterized in that** primers are labelled, in particular with biotin.

3. PCR system according to Claim 1 or 2 for use in detecting human papillomaviruses (HPVs), in particular for discriminating between high-risk and low-risk species of human papillomaviruses (HPVs).

4. PCR system according to Claim 1 or 2 for use in early diagnosis of carcinomas, in particular of precancerous conditions, preferably of cervical cancer.

5. Method for diagnosing infections with human papillomaviruses (HPVs), which method comprises the following steps:
- detection of DNA, comprising
a) controlling the quality of samples
b) testing for low-risk HPVs
c) testing for high-risk HPVs
**characterized in that** the DNA is detected by way of specific regions of HPV DNA, using oligonucleotide probes having at least one of the nucleotide sequences SEQ ID Nos. 25 to 48 and a PCR system according to any of Claims 1 to 4.

6. Method according to Claim 5, **characterized in that** the primers are employed in groups, preferably in groups for common regions and/or for neighbouring regions of the DNA, in particular the HPV DNA.

7. Method according to Claim 5 or 6, **characterized in that** the PCR or PCR-like methods are used as amplification methods.

8. Method according to any of Claims 5 to 7, **characterized in that** the oligonucleotide probes are employed in reverse hybridization.

9. Kit for diagnosing HPV infections, in particular for carrying out a method according to any of Claims 5 to 8, comprising at least one container which has at least one oligonucleotide probe having at least one of the nucleotide sequences SEQ ID Nos. 25 to 48, and a PCR system according to any of Claims 1 to 4 as a means of detecting low-risk HPVs and/or a means of detecting high-risk HPVs.

10. Kit according to Claim 9, further **characterized in that** the at least one oligonucleotide probe is immobilized on a support, in particular on a test strip.

## Revendications

1. Système PCR, comprenant un ensemble d'amorces constitué d'une amorce directe ayant la séquence nucléotidique SEQ ID N° 57 et d'une amorce inverse ayant la séquence nucléotidique SEQ ID N° 58, un ensemble d'amorces constitué d'une amorce directe ayant la séquence nucléotidique SEQ ID N° 59 et d'une amorce inverse ayant la séquence nucléotidique SEQ ID N° 60, un ensemble d'amorces constitué d'une amorce directe ayant la séquence nucléotidique SEQ ID N° 61 et d'une amorce inverse ayant la séquence nucléotidique SEQ ID N° 62, et un ensemble d'amorces constitué d'une amorce directe ayant la séquence nucléotidique SEQ ID N° 63 et d'une amorce inverse ayant la séquence nucléotidique SEQ ID N° 64.

2. Système PCR selon la revendication 1, **caractérisé en ce que** les amorces sont marquées, notamment par de la biotine.

3. Système PCR selon la revendication 1 ou 2 pour utilisation lors de la détection de papillomavirus humains (HPV), en particulier pour permettre une discrimination entre les types à haut risque et les types à faible risque de papillomavirus humains (HPV).

4. Système PCR selon la revendication 1 ou 2 pour utilisation lors de la détection précoce de maladies cancéreuses, en particulier des précancéroses de ces dernières, notamment du cancer du col de l'utérus.

5. Procédé pour le diagnostic d'infections à papillomavirus humains (HPV), qui comprend les étapes suivantes :
- détection de l'ADN
a) par contrôle de la qualité d'échantillons,
b) par un essai portant sur le HPV à faible risque,
c) par un essai portant sur le HPV à haut risque,
**caractérisé en ce que** la détection de l'ADN est effectuée sur des domaines spécifiques de l'ADN du HPV par utilisation de sondes oligonucléotidiques ayant au moins l'une des séquences nucléotidiques SEQ ID N° 25 à 48, et d'un système PCR selon l'une des revendications 1 à 4.

6. Procédé selon la revendication 5, **caractérisé en ce que** les amorces sont utilisées par groupes, de préférence par groupes pour des domaines communs et/ou pour des domaines voisins de l'ADN, en particulier l'ADN du HPV.

7. Procédé selon la revendication 5 ou 6, **caractérisé en ce qu'**on utilise la PCR ou des méthodes analogues à une PCR en tant que procédés d'amplification.

8. Procédé selon l'une des revendications 5 à 7, **caractérisé en ce que** les sondes oligonucléotidiques sont utilisées dans le cadre d'une hybridation inverse.

9. Trousse pour le diagnostic d'infections à HPV, en particulier pour la mise en oeuvre d'un procédé selon l'une des revendications 5 à 8, comprenant au moins un récipient, qui comporte au moins une sonde oligonucléotidique ayant au moins l'une des séquences nucléotidiques SEQ ID N° 25 à 48, et un système PCR selon l'une des revendications 1 à 4, en tant que moyen de détection de HPV à faible risque et/ou en tant que moyen de détection de HPV à haut risque.

10. Trousse selon la revendication 9, **caractérisée en outre en ce que** la ou les sondes oligonucléotidiques sont immobilisées sur un support, en particulier une bandelette d'essai.
